Europäisches Patentamt

European Patent Office

Office européen des brevets

(19)

(11) Veröffentlichungsnummer: **0 048 893**
**A1**

(12) # EUROPÄISCHE PATENTANMELDUNG

(21) Anmeldenummer: 81107314.7

(22) Anmeldetag: 16.09.81

(51) Int. Cl.³: **C 07 C 2/24, C 07 C 41/06,**
**C 07 C 43/04, B 01 J 31/28,**
**B 01 J 31/32**

(30) Priorität: 27.09.80 DE 3036481

(43) Veröffentlichungstag der Anmeldung: 07.04.82
Patentblatt 82/14

(84) Benannte Vertragsstaaten: **BE DE FR GB IT NL**

(71) Anmelder: **EC ERDÖLCHEMIE GMBH, Postfach 75 2002,**
**D-5000 Köln 71 (DE)**

(72) Erfinder: **Köhler, Hans-Dieter, Dr.,**
**Stürzelbergerstrasse 71, D-4047 Dormagen 5 (DE)**
Erfinder: **Scheef, Hans-Volker, Goethestrasse 69,**
**D-4047 Dormagen (DE)**
Erfinder: **Schleppinghoff, Bernhard, Dr., Adolf Kolping**
**Strasse 5, D-4047 Dormagen 1 (DE)**

(74) Vertreter: **Mann, Volker, Dr. et al, c/o Bayer**
**Aktiengesellschaft Zentralbereich Patente Marken und**
**Lizenzen, D-5090 Leverkusen-Bayerwerk (DE)**

(54) Verfahren zur gemeinsamen Herstellung von C4-Oligomeren und Alkyl-tert.-butylethern.

(57) $C_4$-Oligomere und Alkyl-tert.-butylether können durch Umsetzung von Isobuten mit Alkanolen in flüssiger Phase bei 20 bis 140°C hergestellt werden, wobei gleichzeitig die Oligomerisierung und die Etherbildung ablaufen. Hierbei wird in Gegenwart eines mikroporösen oder gelförmigen, sauren Kationenaustauschers in der $H^+$-Form gearbeitet, der 0,05 bis 5 g eines oder mehrerer Metalle der VII. und/oder VIII. Nebengruppe des Periodensystems der Elemente in elementarer Form pro Liter trockenen Kationenaustauscher enthält. Der Kationenaustauscher hat einen Vernetzungsgrad im Bereich von 2 bis 65% und eine spezifische Oberfläche im Bereich von 5 bis 750 m²/g trockenes Austauscherharz.

EP 0 048 893 A1

0048893

EC Erdölchemie GmbH          Köln-Worringen
                             Gai/kl-c


Verfahren zur gemeinsamen Herstellung von $C_4$-Oligomeren
und Alkyl-tert.-butylethern


Die vorliegende Erfindung betrifft ein Verfahren zur
gleichzeitigen Herstellung in einem Reaktor von $C_4$-
Oligomeren und Alkyl-tert.-butylethern aus Isobuten
oder Isobuten enthaltenden Gemischen und Alkanolen
in Gegenwart spezieller Ionenaustauscher.

Bei der Aufarbeitung von $C_4$-Fraktionen, wie sie z.B.
bei Crackprozessen anfallen, wird im allgemeinen zunächst das vorhandene Butadien ganz oder weitgehend
entfernt. Es hinterbleibt dann ein Gemisch, das im
wesentlichen Buten-(1), Buten-(2), Isobuten und gesättigte $C_4$-Kohlenwasserstoffe enthält. Solche Gemische
werden im allgemeinen aufgearbeitet, indem man in solchen Gemischen das Isobuten zu technisch interessanten Produkten umsetzt und diese abtrennt. Das dann
verbleibende, im wesentlichen Buten-(1), Buten-(2)
und gesättigte $C_4$-Kohlenwasserstoffe enthaltende Gemisch kann beispielsweise zur Herstellung von reinem
Buten-(1), einem hochwertigen Comonomeren bzw. Homomonomeren oder nach Umsatz mit Isobutan als Alkylatbenzin verwendet werden. Es ist dabei vorteilhaft, wenn


EC 118 -Ausland

das nach der Umsetzung von Isobuten und Abtrennung der Umsetzungsprodukte verbleibende Gemisch einen hohen Anteil an Buten-(1) aufweist. Bei der Umsetzung von Isobuten soll also möglichst wenig Buten-(1) in Buten-(2) umgelagert werden.

Die Umsetzung von Isobuten zu technisch interessanten Folgeprodukten kann z.B. darin bestehen, daß man das Isobuten in Schlaufenreaktoren in Gegenwart von sauren Kationenaustauschern zu Dimeren, Trimeren und höheren Oligomeren umsetzt und diese Oligomere abtrennt (siehe Erdöl und Kohle, Erdgas, Petrochemie 19, (7), 497 bis 500, (1966)). Dieses einfache und mit hohem Umsatz ablaufende Verfahren hat jedoch den Nachteil, das mindestens 50 % des im Ausgangsgemisch vorhandenen Buten-(1) zu Buten-(2) umgelagert wird (siehe Erdöl und Kohle, 27, (5), 245 (1974)). Die Oligomeren des Isobutens können auf verschiedene Weise weiterverwendet werden. So sind insbesondere Diisobuten (DIB) und Triisobuten (TIB), auch in hydrierter Form, wichtige, oktanzahlerhöhende Zusätze zu Kraftstoffen für Ottomotoren (siehe Hydrocarb. Proc., April 1973, S. 171 bis 173). DIB und seine Codimeren lassen sich auch gut durch Oxosynthese in Isononylalkohole überführen, die nach Überführung in die Isononylphthalate großtechnisch als Weichmacher, z.B. für Polyvinylchlorid verwendet werden (siehe Erdöl und Kohle, 27 (2), S. 77 bis 82 (1974)). Weiterhin können DIB und TIB in hydrierter Form als aromatenfreie Isoparaffine als Lösungs- und Verdünnungsmittel verwendet werden (siehe BP-Firmenschrift: Lösungsmittel, Spezialbenzine, Testbenzine, Aromaten; Ausgabe 1973).

EC 118

- 3 -

Die Umsetzung von Isobuten zu technisch interessanten Folgeprodukten kann z.B. auch darin bestehen, daß man das Isobuten mit Alkoholen unter Bildung von Alkyl-tert.-butylethern umsetzt. Insbesondere ist hier die Veretherung mit Methanol unter Bildung von Methyl-tert.-butylether (MTBE) von Interesse. Als Katalysator für diese Veretherungsreaktion verwendet man im allgemeinen saure Kationenaustauscher. Die gebildeten Ether, insbesondere MTBE, werden von den verbleibenden $C_4$-Kohlenwasserstoffen und gegebenenfalls von überschüssigem Alkohol abgetrennt (siehe z.B. Hydr. Proc. 1977, (11), 185; Oil and Gas Journal, Jan. 1 (1976), 76 bis 77; Hydr. Proc. 1977, (12), 98 bis 102).

MTBE und Ethyl-tert.-butylether können als Oktanzahlerhöhender Zusatz zu Kraftstoffen für Ottomotoren verwendet werden (siehe Hydrocarb. Proc., Feb. 1980, S. 57 bis 59). Höhere Alkyl-tert.-butylether können als Lösungs- und Verdünnungsmittel verwendet werden (siehe NL-Patentanmeldung 74/10 872 und DE-OS 26 20 011).

Bisher ist kein Verfahren bekannt geworden, gemäß dem man aus Isobuten $C_4$-Oligomere und Alkyl-tert.-butylether gleichzeitig in einem Reaktor herstellen kann. Bisher ist man davon ausgegangen, daß die Gegenwart von Alkoholen, insbesondere von Methanol, die $C_4$-Oligomerisierung teilweise inhibiert (siehe z.B. DE-OS 2 853 769, gemäß der bei der MTBE-Herstellung die Bildung von Spuren von $C_8$-Oligomeren erwähnt wird, eine gleichzeitige Herstellung von MTBE und $C_8$-Oligomeren in technischem Maßstab jedoch nicht möglich ist).

EC 118

Es wurde nun ein Verfahren zur Herstellung von $C_4$-Oligomeren und Alkyl-tert.-butylethern durch Umsetzung von Isobuten und Alkoholen in Gegenwart von Kationenaustauschern gefunden, das dadurch gekennzeichnet ist, daß man die Umsetzung von Isobuten zu $C_4$-Oligomeren und die Umsetzung von Isobuten mit Alkanolen zu Alkyl-tert.-butylethern gleichzeitig in einem Reaktor in flüssiger Phase bei 20 bis 140 °C und in Gegenwart eines makroporösen oder gelförmigen, sauren Kationenaustauschers in der $H^+$-Form durchführt, der 0,05 bis 5 g eines oder mehrerer Metalle der 7. und/oder 8. Nebengruppe des Periodensystems der Elemente in elementarer Form, pro Liter trocknen Kationenaustauscher, enthält, wobei der Kationenaustauscher einen Vernetzungsgrad im Bereich 2 bis 65 % und eine spezifische Oberfläche im Bereich 5 bis 750 m²/g trockenes Austauscherharz aufweist.

Das für das erfindungsgemäße Verfahren benötigte Isobuten kann in verschiedner Form eingesetzt werden. Es kann beispielsweise in reiner oder nahezu reiner Form, aber auch in Form von isobutenhaltigen Gemischen eingesetzt werden. Als isobutenhaltige Gemische kommen insbesondere solche in Frage, die keine oder nur sehr wenig Bestandteile enthalten, die das erfindungsgemäße Verfahren stören können. Solche störenden Bestandteile können beispielsweise Verbindungen sein, die Dreifachbindungen enthalten. Solche Verbindungen sind vorzugsweise zu weniger als 1 Gew.-% in isobutenhaltigen Gemischen

EC 118

enthalten, die in das erfindungsgemäße Verfahren eingesetzt werden sollen. Bevorzugt sind solche Verbindungen zu weniger als 0,1 Gew.-% in den isobutenhaltigen Gemischen enthalten. Neben Isobuten können in den isobutenhaltigen Gemischen beispielsweise gesättigte Kohlenwasserstoffe, einfach ungesättigte Kohlenwasserstoffe und gegebenenfalls sonstige, im erfindungsgemäßen Verfahren nicht störende Verbindungen, enthalten sein. Vorzugsweise enthält ein in das erfindungsgemäße Verfahren einsetzbares Gemisch zwischen 10 und 80 Gew.-% Isobuten. Gemische, die zwischen 20 und 60 Gew.-% Isobuten enthalten sind zum Einsatz in das erfindungsgemäße Verfahren besonders bevorzugt.

Wenn in das erfindungsgemäße Verfahren reines Isobuten oder isobutenhaltige Gemische mit hohem Isobutengehalt, beispielsweise mit einem Isobutengehalt von über 80 Gew.-%, eingesetzt werden sollen, so ist es im allgemeinen vorteilhaft,besondere Maßnahmen zu ergreifen, um die Reaktionswärme in ausreichendem Maß abführen zu können. Beispielsweise kann man in solchen Fällen einen Teil der Reaktionsprodukte in die Reaktion zurückführen und so das Reaktionsgemisch verdünnen. Man kann auch dem Einsatzgemisch Lösungsmittel, z.B. Butane, zufügen oder durch entsprechende Dimensionierung des Reaktors und seiner Kühleinrichtungen die Wärmeabfuhr verbessern.

Besonders geeignet zum Einsatz in das erfindungsgemäße Verfahren sind Gemische geradkettiger und verzweigter

EC 118

Butane und Butene. Solche Gemische fallen beispielsweise beim thermischen oder katalytischen Cracken von Mineralölfraktionen an (sogenannte $C_4$-Schnitte), die nach Entfernung des darin enthaltenen Butadiens als Einsatzgemische für das erfindungsgemäße Verfahren besonders geeignet sind. Typische Zusammensetzungen für solche vom Butadien befreiten $C_4$-Schnitte sind beispielsweise:

|  | aus der thermischen Crackung (Steamcracker) | aus der katalytischen Crackung (Catcracker) |
| --- | --- | --- |
| n-Butan | 6 bis 8 Gew.-% | etwa 10 Gew.-% |
| i-Butan | 2 bis 3 Gew.-% | etwa 34 Gew.-% |
| i-Buten | 44 bis 49 Gew.-% | etwa 15 Gew.-% |
| Buten-(1) | 24 bis 28 Gew.-% | etwa 13 Gew.-% |
| Buten-(2) | 19 bis 21 Gew.-% | etwa 28 Gew.-% |

In das erfindungsgemäße Verfahren einsetzbare Alkanole sind beispielsweise solche mit 1 bis 8 Kohlenstoffatomen, wie Methanol, Ethanol, Propanole, Butanole, Hexanole, Heptanole und Oktanole. Bevorzugt werden Alkanole mit 1 bis 6 Kohlenstoffatomen eingesetzt, besonders bevorzugt solche mit 1 bis 4 Kohlenstoffatomen, ganz besonders bevorzugt Methanol. Aus dem Alkanol bildet sich im erfindungsgemäßen Verfahren durch Umsetzung mit Isobuten der entsprechende Alkyl-tert.-butylether. So entsteht beispielsweise aus Isobuten und Methanol Methyl-tert.-butylether (MTBE).

EC 118

Das Mengenverhältnis von eingesetztem Isobuten zu Alkanol kann beim erfindungsgemäßen Verfahren in sehr weiten Grenzen variiert werden. So kann das Molverhältnis von Isobuten zu Alkanol beispielsweise im Bereich von 0,5 bis $5 \times 10^5$ zu 1 liegen. Bevorzugt liegt dieses Molverhältnis im Bereich 0,9 bis $5 \times 10^4$ zu 1, besonders bevorzugt im Bereich 1 bis $5 \times 10^3$ zu 1. Mit dem Molverhältnis Isobuten zu Alkanol läßt sich die Zusammensetzung des Reaktionsproduktes steuern. Wenn im Verhältnis zu Isobuten wenig Alkanol eingesetzt wird, so entstehen aus Isobuten überwiegend $C_4$-Oligomere. Wenn im Verhältnis zu Isobuten viel Alkanol eingesetzt wird, so entsteht aus Isobuten überwiegend der dem Alkanol entsprechende Alkyl-tert.-butylether.

Das erfindungsgemäße Verfahren wird in flüssiger Phase bei Temperaturen im Bereich 20 bis 140°C durchgeführt. Beispielsweise sind auch Temperaturen im Bereich 20 bis 120°C geeignet. Vorzugsweise arbeitet man im Bereich 25 bis 100°C, besonders bevorzugt im Bereich 30 bis 80°C. Ganz besonders bevorzugt sind Temperaturen zwischen 35 und 60°C. Der Druck wird so gewählt, daß bei gegebener Temperatur zumindest ein Teil der Ausgangs- und Reaktionsprodukte in flüssiger Phase vorliegt. Dies kann im allgemeinen erreicht werden, wenn man Drucke im Bereich 2 bis 100 bar, vorzugsweise im Bereich 3 bis 30 bar anwendet. Hohe Drucke und tiefe Temperaturen (innerhalb der angegebenen Bereiche) wirken sich auf die Etherbildung günstig aus.

EC 118

Im allgemeinen ist die Bildung der $C_4$-Oligomeren und des Alkyl-tert.-butylethers innerhalb 0,1 bis 5 Stunden beendet. Vorzugsweise beträgt die Reaktionszeit 0,2 bis 3 Stunden, besonders bevorzugt 0,3 bis 1 Stunden.

Es ist ein wesentliches Merkmal des erfindungsgemäßen Verfahrens, daß man es durchführt in Gegenwart eines makroporösen oder gelförmigen, sauren Kationenaustauschers in der $H^+$-Form, der 0,05 bis 5 g eines oder mehrerer Metalle der 7. und/oder 8. Nebengruppe des Periodensystems der Elemente in elementarer Form, pro Liter trockenen Kationenaustauscher, enthält, wobei der Kationenaustauscher einen Vernetzungsgrad im Bereich 2 bis 65 % mit einer spezifischen Oberfläche im Bereich von 5 bis 750 m²/g trockenes Austauscherharz aufweist.

Erfindungsgemäß einzusetzende Kationenaustauscher sind beispielsweise durch Copolymerisation von Vinylmonomeren mit Divinylvernetzern in Gegenwart von Lösungsmitteln herstellbar, wobei die Lösungsmittel die Monomeren zu lösen vermögen, aber keine Quellwirkung für die entstehenden Polymeren zeigen (siehe z.B. DE-AS 11 13 570, US-PS 3 586 646). Als Vinylmonomere können z.B. Styrol oder Acrylsäureester eingesetzt werden. Als Divinylvernetzer kommt z.B. Divinylbenzol in Frage. Der Vernetzungsgrad ist abhängig von der Menge des Divinylvernetzers und liegt vorzugsweise im Bereich von 8 bis 25 %. Als Vernetzungsgrad wird hier die Menge des Divinylvernetzers bezogen auf die Gesamtmenge an Comonomeren verstanden.

EC 118

Als saure Gruppen können die Kationenaustauscher beispielsweise Carboxylgruppen oder Sulfonsäuregruppen enthalten. Carboxylgruppen können z.B. durch Verseifung von Acrylester-Gruppen, Sulfonsäuregruppen z.B. durch nachträgliche Sulfonierung eingeführt werden.

Bevorzugt werden stark saure, Sulfonsäuregruppen enthaltende Styrol-Divinylbenzol-Polymerisate eingesetzt. Solche Kationenaustauscher sind unter verschiedenen Bezeichnungen im Handel erhältlich.

Vorzugsweise weisen die erfindungsgemäß einzusetzenden Kationenaustauscher eine spezifische Oberfläche im Bereich 50 bis 250 m²/g auf. Der mittlere Porenradius der Kationenaustauscher kann beispielsweise in den Grenzen von 50 bis 1200 Å variieren. Vorzugsweise liegt der mittlere Porenradius im Bereich 70 bis 500 Å.

Wenn die Kationenaustauscher als Perlpolymerisate eingesetzt werden, können sie beispielsweise Korngrößen von 0,1 bis 2 mm aufweisen. Wenn sie als Pulverharze zum Einsatz gelangen können sie beispielsweise Korngrößen von 10 bis 100 µ aufweisen.

In das erfindungsgemäße Verfahren werden die Kationenaustauscher in der $H^+$-Form eingesetzt, nachdem sie mit 0,05 bis 5 g (bezogen auf einen Liter trockenen Kationenaustauscher) eines oder mehrerer Metalle der 7. und/oder 8. Nebengruppe des Periodensystems der Elemente (wie beispielsweise abgedruckt in CRC-Handbook of Chemistry and Physics, 60. Auflage, 1979 - 1980, heraus-

EC 118

gegeben von R.C. Weast, CRC-Press, Bota Raton, Florida, USA) in elementarer Form belegt worden sind. Als Metalle der 7. oder 8. Nebengruppe des Periodensystems kommen beispielsweise Mangan, Rhenium, Eisen, Kobalt, Nickel, Ruthenium, Rhodium, Palladium, Osmium, Iridium und/oder Platin in Frage. Vorzugsweise ist der Kationenaustauscher mit 0,1 bis 2 g (pro Liter trockenen Kationenaustauscher) mit eines oder mehrerer dieser Metalle belegt worden. Vorzugsweise kommen als Metalle Rhenium, Palladium, Platin und Nickel in Frage, jeweils für sich alleine. Ganz besonders bevorzugt ist Palladium, Platin und Nickel. Wenn der Kationenaustauscher mit mehreren Metallen belegt worden ist, so beträgt die Gesamtmenge der Metalle 0,05 bis 5 g, vorzugsweise 0,1 bis 2 g pro Liter trockenen Kationenaustauscher.

Die Belegung der Kationenaustauscher mit dem oder den Metallen kann beispielsweise erfolgen, indem man zunächst das oder die gewünschten Metalle in Form einer Lösung, z.B. in Wasser, von nicht komplexen, kationischen Salzen ansatzweise mit dem Kationenaustauscher in der $H^+$-Form in an sich bekannter Weise zusammenbringt. Solche Metallsalze können beispielsweise Chloride, Bromide, Nitrate, Sulfate und/oder Acetate sein.

Die Menge des Salzes wird so gewählt, daß nach der weiteren Behandlung, insbesondere der Reduzierung, sich die gewünschte Menge Metall auf dem Kationenaustauscher befindet. Die einzusetzende Salzmenge kann gegebenenfalls durch einfaches Probieren ermittelt werden.

EC 118

Es kann vorteilhaft sein, die beim Zusammenbringen des oder der Metallsalze mit dem Kationenaustauscher freiwerdende Säure zu neutralisieren. Diese Neutralisation kann während oder nach dem Zusammenbringen des oder der Metallsalze mit den Kationenaustauscher erfolgen. Für diese Neutralisation sind alkalisch reagierende Verbindungen, gegebenenfalls in Form einer wäßrigen Lösung, geeignet, wie Natriumhydroxid, Kaliumhydroxid, Ammoniak, Natriumcarbonat, Kaliumcarbonat und/oder Ammoniumcarbonat. Nach dem Zusammenbringen von Metallsalz(en) und Kationenaustauscher und gegebenenfalls nach der Neutralisation wird der Kationenaustauscher im allgemeinen neutral gewaschen und anschließend getrocknet, z.B. bei erhöhter Temperatur und gegebenenfalls bei vermindertem Druck. Geeignete Trocknungsbedingungen sind beispielsweise 100°C und Wasserstrahlpumpenvakuum für 24 Stunden.

Zur Überführung der so aufgebrachten Metalle in den elementaren Zustand kann der so vorbehandelte Kationenaustauscher mit Wasserstoff behandelt werden. Diese Behandlung kann beispielsweise bei 2 bis 50 bar, vorzugsweise bei 20 bis 30 bar und bei einer Temperatur im Bereich von beispielsweise 50 bis 140°C, vorzugsweise 80 bis 120°C, durchgeführt werden.

Der erfindungsgemäß einzusetzende Kationenaustauscher kann bei diskontinuierlicher Arbeitsweise beispielsweise in Mengen von 1 bis 50 Gewichtsteilen Reaktionsgemisch, bezogen auf 1 Gewichtsteil Kationenaustauscher eingesetzt werden. Bevorzugt werden 5 bis 50, besonders

EC 118

bevorzugt 10 bis 30 Gewichtsteile Reaktionsgemisch bezogen auf 1 Gewichtsteil Kationenaustauscher bei diskontinuierlicher Arbeitsweise eingesetzt. Bei kontinuierlicher Arbeitsweise kann der Kationenaustauscher beispielsweise in solchen Mengen eingesetzt werden, daß sich eine Belastung a von 0,1 bis 10, vorzugsweise von 1 bis 5 ergibt. a bedeutet dabei

$$\frac{\text{Kohlenwasserstoffe im Einsatz } \lceil \text{g} \rfloor}{\text{Kationenaustauscher } \lceil \text{g} \rfloor \cdot \text{Zeit } \lceil \text{h} \rfloor}$$

Nach Beendigung der Reaktion kann man das Reaktionsgemisch in an sich bekannter Weise aufarbeiten. Vorzugsweise trennt man im Falle einer diskontinuierlichen Arbeitsweise zunächst den Kationenaustauscher ab, z.B. durch Dekantieren oder Zentrifugieren. Der Kationenaustauscher kann dann mindestens 20 mal wiederverwendet werden, ohne eine nennenswerte Abnahme der Aktivität. Von dem vom Kationenaustauscher befreiten Reaktionsgemisch kann man einen Teil erneut in die erfindungsgemäße Umsetzung zurückführen. Durch die Rückführung von Reaktionsprodukt, beispielsweise im Gewichtsverhältnis Rückführung zu Entnahme von 0,1 bis 10:1, kann man innerhalb der $C_4$-Oligomeren eine Erhöhung des Anteils höherer Oligomerer und eine Erniedrigung des Anteils niedriger Oligomerer erreichen oder, insbesondere beim Einsatz von reinem Isobuten oder isobutenhaltigen Gemischen mit hohem Isobutengehalt die Reaktionswärme besser abführen. Falls eine Rückführung von Reaktionsprodukt durchgeführt wird, erfolgt

EC 118

diese vorzugsweise im Gewichtsverhältnis von Rückführung zu Entnahme von 1:1 bis 3:1.

Aus dem entnommenen Reaktionsprodukt kann man sowohl die $C_4$-Oligomeren, z.B. Diisobuten (DIB), Triisobuten (TIB) und/oder Tetraisobuten, als auch den Alkyl-tert.-butylether und gegebenenfalls vorhandenes unumgesetztes Alkanol abtrennen, z.B. durch Destillation, und getrennt oder gemeinsam einer der eingangs genannten Verwendungen zuführen. Das entnommene Reaktionsprodukt kann auch nach Durchlaufen eines Debutanizers als Vergaserkraftstoff verwendet werden. Sofern das Reaktionsprodukt unumgesetztes Alkanol enthält, ist es bevorzugt, dieses abzutrennen und erneut in das erfindungsgemäße Verfahren einzusetzen.

In einer diskontinuierlichen Variante kann das erfindungsgemäße Verfahren beispielsweise wie folgt durchgeführt werden:

In einem geschlossenen Reaktor wird das zu oligomerisierende und zu verethernde Isobuten, gegebenenfalls im Gemisch mit einem oder mehreren weiteren Olefinen und/oder inerten Kohlenwasserstoffen mit dem Alkanol im vorgewählten molaren Verhältnis in Gegenwart eines der oben beschriebenen Kationenaustauscher im Verhältnis von 10 bis 30 Gew.-Teile Reaktionsgemisch pro Gew.-Teil Kationenaustauscher versetzt und etwa

EC 118

0048893

1/2 bis 5 Stunden unter einem Druck von 3 bis 30 bar und bei einer Temperatur von 30 bis 80°C gerührt oder geschüttelt, bis die Oligomerisierung und die Veretherung abgeschlossen ist. Danach wird das Reaktionsgemisch entspannt und der Kationenaustauscher abgetrennt. Das Reaktionsgefäß kann dann unter Wiederverwendung des Kationenaustauschers mit einem neuen Reaktionsansatz mindestens 20 mal beschickt werden, ohne daß eine wesentliche Abnahme der Aktivität des Kationenaustauschers festgestellt wird.

In einer kontinuierlichen Variante des erfindungsgemäßen Verfahrens kann beispielsweise wie folgt verfahren werden:

Einer der oben beschriebenen Kationenaustauscher wird in ein temperierbares Stahl- oder Glasgefäß, beispielsweise eines oder mehrere temperierbare Reaktionsrohre eingefüllt. Das Isobuten oder ein dieses enthaltendes Gemisch und das Alkanol werden einzeln oder als Gemisch von unten nach oben oder von oben nach unten, beispielsweise in der Rieselphase, vorzugsweise in der Flüssigphase, durch das Austauscherbett geführt. Vorzugsweise arbeitet man hier mit einer konstanten Fließgeschwindigkeit. Durch Variation des Druckes, der Temperatur und der Belastung des Kationenaustauschers bzw. der Verweilzeit können durch einfaches Ausprobieren die für das Isobuten oder es enthaltende Gemische günstigsten Bedingungen gefunden werden. Der

EC 118

den Reaktor verlassende Produktstrom wird gesammelt und der Gehalt an Oligomeren und Ethern kann darin beispielsweise durch gaschromatographische Analyse bestimmt werden. Die erhaltenen Produkte können wie zuvor beschrieben aufgearbeitet und verwendet werden.

Das erfindungsgemäße Verfahren weist eine Reihe von überraschenden Vorteilen auf, von denen die wichtigsten im folgenden aufgezählt sind:

1) $C_4$-Oligomere und Alkyl-tert.-butylether können in einem Reaktionsgefäß gleichzeitig hergestellt werden, wobei die Art der $C_4$-Oligomeren und das Verhältnis von $C_4$-Oligomeren zu Alkyl-tert.-butylethern in weiten Grenzen variiert und so den jeweiligen Bedürfnissen angepaßt werden kann.

2) Eine Inhibierung der $C_4$-Oligomerisierung durch die Gegenwart von Alkanol wird nicht beobachtet.

3) Im Vergleich zu der separaten Herstellung von $C_4$-Oligomeren auf übliche Weise, bei der in den erhaltenen Oligomeren etwa 60 bis 66 Gew.-% Diisobuten (z.B. Trimethylpenten) vorliegt, können erfindungsgemäß $C_4$-Oligomere mit einem Diisobuten-Anteil von etwa 80 bis 85 % erhalten werden unter gleichzeitiger Erniedrigung des Gehaltes unerwünschter Codimerer (z.B. Dimethylhexen).

EC 118

4) Wenn n-butenhaltige Isobutenmischungen eingesetzt werden, so wird bereits beim Einsatz geringer Mengen an Alkanol nur eine geringe Umlagerung von Buten-(1) zu Buten-(2) beobachtet. Das wertvolle Buten-(1) bleibt in solchen Fällen weitgehend erhalten.

5) Im Kationenaustauscherbett wurden keine nennenswerten Temperaturspitzen, die zu Kontaktschädingungen führen könnten, beobachtet. Die Reaktionswärme kann über die Kationenaustauscherpackung gleichmäßig in das sie umgebende Medium und von dort gegebenenfalls nach außen abgeführt werden.

6) Die Oligomerisierung und Veretherung setzt bereits bei niedrigen Temperaturen ein, so daß das Anfahren und Abfahren eines Reaktors problemlos durchgeführt werden kann.

7) Das zugesetzte Alkanol kann quantitativ verethert werden. Im allgemeinen können Trennoperationen zur Abtrennung von Alkanolen aus dem Reaktionsprodukt entfallen. Gemäß der DE-OS 2 853 769 ist hier eine aufwendige Azetropdestillation unter Durck durchzuführen.

8) Die Bildung von höheren Ethern, wie $C_5$Ethern, konnte praktisch nicht beobachtet werden.

Die folgenden Beispiele erläutern das erfindungsgemäße Verfahren, ohne es einzuschränken.

EC 118

## Beispiele

### Beispiel 1 (Herstellung eines erfindungsgemäß zu verwendenden Kationenaustauschers)

Entsprechend der DE-PS 1 113 570, Beispiel 3, wurde ein makroporöser Kationenaustauscher hergestellt. Der wasserfeuchten $H^+$-Form dieses Kationenaustauschers wurde im Batch so viel Palladiumacetat angeboten, daß 0,75 g Palladium pro Liter trockenes Harz nach Reduktion mit Wasserstoff auf dem Kationenaustauscher vorhanden waren. Die bei der Behandlung mit Palladiumacetat freigesetzte Säure wurde mit 1 gew.-%iger Natronlauge neutralisiert. Anschließend wurde der neutral gewaschene Kationenaustauscher 24 Stunden bei 100°C im Vakuum einer Wasserstrahlpumpe getrocknet. Das auf dem Kationenaustauscher befindliche Palladium wurde dann zwischen 90 und 100°C mit Wasserstoff bei einem Druck von 20 bis 25 bar innerhalb von 48 Stunden zum Metall reduziert.

### Beispiel 2

Als Oligomerisierungs- und Veretherungsapparatur fand ein temperierbarer Durchlaufreaktor mit einem lichten Reaktordurchmesser von 20 mm Anwendung. Es wurde so viel des gemäß Beispiel 1 hergestellten Kationenaustauschers eingefüllt, daß sich in Relation zu der eingesetzten Flüssigkeitsmenge eine zuvor definierte Belastung des Kationenaustauschers von a = 1,5 ergab. Zur

EC 118

Temperaturkontrolle war der Reaktor mit mehreren Temperaturmeßstellen in Abständen von jeweils 100 mm ausgerüstet. Die Reaktionstemperatur wurde bei 40°C gehalten. Der Reaktor war über eine Druckhaltung druckgeregelt. Der Druck wurde auf 15 bar gehalten. Das eingesetzte Kohlenwasserstoffgemisch (siehe Tabelle 1) wurde mit Methanol in einer Mischkammer vor dem Reaktor vermischt. Das den Reaktor verlassende Reaktionsprodukt wurde in einem gekühlten Abscheider zwischengelagert bevor es anschließend destillativ aufgearbeitet wurde. Dabei wurde in einer ersten Destillationskolonne am Kopf ein isobutenfreies Buten-(1)-reiches $C_4$-Kohlenwasserstoffgemisch (sog. Raffinat II) und am Sumpf Methyl-tert.-butylether und $C_4$-Oligomere entnommen. Aus diesem Sumpfprodukt wurde in einer nachgeschalteten Destillation Methyl-tert.-butylether von den $C_4$-Oligomeren getrennt. Die Analysen wurden gaschromatographisch durchgeführt. Die Probenentnahme zur gaschromatographischen Bestimmung der Endprodukte erfolgte nach einer Reaktionszeit von 60 Stunden bei konstanter Kationenaustauscherbelastung. Die Zusammensetzung des Einsatz- und Endproduktes und sonstige Kenndaten der Reaktion sind aus Tabelle 1 ersichtlich.

Beispiel 3 (Vergleichsbeispiel)

Entsprechend der DE-PS 1 113 570, Beispiel 3, wurde ein makroporöser Kationenaustauscher hergestellt. Das so erhaltene wasserfeuchte Ionenaustauscherharz wurde zur Entfernung des Wassers im Trockenschrank bei 90 -

EC 118

100°C und 15 Torr über Nacht getrocknet. Der so erhaltene trockene Kationenaustauscher, der kein Metall der 7. und/oder 8. Nebengruppe des Periodensystems enthielt, wurde in eine Beispiel 2 entsprechende Umsetzung eingesetzt, wobei das Einsatzprodukt jedoch kein Methanol enthielt. Die Zusammensetzung des Einsatz- und Endproduktes und sonstige Kenndaten der Reaktion sind aus Tabelle 1 ersichtlich.

### Beispiele 4 bis 6

Die Versuche wurden wie Beispiel 2 beschrieben durchgeführt, jedoch wurde ein handelsüblicher Kationenaustauscher eingesetzt, der gemäß der in Beispiel 1 beschriebenen Arbeitsweise mit 1,25 g Palladium pro Liter getrocknetem Kationenaustauscher belegt worden war und wobei wechselnde Mengen Methanol, bezogen auf Isobuten, eingesetzt wurden. Die Zusammensetzung der Einsatz- und Endprodukte und sonstige Kenndaten der Reaktionen sind aus Tabelle 1 ersichtlich.

EC 118

Tabelle 1

| Beispiel Kationenaustauscher | [Nr] [Bez] | 2 H$^+$/Pd | 3 H$^+$ |
|---|---|---|---|
| Einsatzprodukt | [g/h] | 100 | 100 |
| Isobuten | [Gew.-%] | 46,4 | 47,0 |
| n-Butene | [Gew.-%] | 41,1 | 41,1 |
| Buten-1 | [g] | 24,25 | 24,87 |
| Buten-2 | [g] | 16,85 | 16,23 |
| Methanol | [g/h] | 0,4 | - |
| Reaktionsprodukt | [g/h] | 100,5 | 100,0 |
| C$_4$ | [Gew.-%] | 38,2 | 39,6 |
| i-Buten | [Gew.-%] | 0,2 | 0,1 |
| n-Butene | [Gew.-%] | 25,8 | 27,0 |
| Buten-1 | [g] | 15,0 | 3,8 |
| Buten-2 | [g] | 10,8 | 23,2 |
| Umsatz i-Buten | [%] | 99,6 | 99,8 |
| C$_8$ | [Gew.-%] | 41,0 | 33,8 |
| Dimer | [%] | 76,0 | 66,0 |
| Codi I | [%] | 1,2 | 1,2 |
| Codi im Dimer | [%] | 2,8 | 3,8 |
| Codi II | [%] | 19,0 | 28,1 |
| C$_{12}$ | [Gew.-%] | 17,1 | 21,5 |
| C$_{16}$ | [Gew.-%] | 2,5 | 4,9 |
| C$_{20}$ | [Gew.-%] | 0,2 | 0,2 |
| MTBE | [Gew.-%] | 1,0 | <0,1 |
| C$_9$-Ether | [Gew.-%] | <0,01 | <0,1 |
| Methanol | [Gew.-%] | <0,01 | <0,01 |

Codi I bedeutet: Umsetzungsprodukte von Isobuten und n-Buten, die niedriger als Diisobuten sieden.
Codi II bedeutet: Umsetzungsprodukte von Isobuten und n-Buten, die höher als Diisobuten sieden.
MTBE bedeutet: Methyl-tert.-butylether

EC 118

Tabelle 1 (Fortsetzung)

| Beispiel | [Nr] | 4 H$^+$/Pd | 5 H$^+$/Pd | 6 H$^+$/Pd |
|---|---|---|---|---|
| Kationenaustauscher | [Bez] | | | |
| Einsatzprodukt | [g/h] | 100 | 100 | 100 |
| Isobuten | [Gew.-%] | 46,4 | 44,2 | 47,2 |
| n-Butene | [Gew.-%] | 41,1 | 41,1 | 41,1 |
| Buten-1 | [g] | 24,87 | 25,19 | 25,19 |
| Buten-2 | [g] | 16,23 | 15,90 | 15,90 |
| Methanol | [g/h] | 10,0 | 25,24 | 26,95 |
| Reaktionsprodukt | [g/h] | 110,0 | 125,24 | 127,0 |
| C$_4$ | [Gew.-%] | 47,0 | 41,4 | 38,7 |
| i-Buten | [Gew.-%] | 0,1 | 0,1 | 0,1 |
| n-Butene | [Gew.-%] | 34,5 | 28,9 | 26,1 |
| Buten-1 | [g] | 23,1 | 22,7 | 19,9 |
| Buten-2 | [g] | 14,8 | 13,5 | 13,2 |
| Umsatz i-Buten | [%] | 99,8 | 99,7 | 99,7 |
| C$_8$ | [Gew.-%] | 24,9 | 2,2 | 1,0 |
| Dimer | [%] | 86,8 | 86,8 | 90,0 |
| Codi I | [%] | 0,8 | 1,0 | <0,1 |
| Codi im Dimer | [%] | 2,4 | 2,0 | <0,1 |
| Codi II | [%] | 9,6 | 9,8 | 10,0 |
| C$_{12}$ | [Gew.-%] | 3,3 | 0,3 | 0,2 |
| C$_{16}$ | [Gew.-%] | 0,2 | <0,1 | <0,1 |
| C$_{20}$ | [Gew.-%] | <0,1 | <0,1 | <0,1 |
| MTBE | [Gew.-%] | 24,5 | 55,7 | 59,3 |
| C$_9$-Ether | [Gew.-%] | <0,1 | <0,1 | <0,1 |
| Methanol | [Gew.-%] | <0,1 | <0,1 | <0,1 |

Codi I, Codi II und MTBE haben die vorstehend angegebene
Bedeutung.

EC 118

<u>Beispiele 7 bis 9</u>

Es wurde verfahren wie in Beispiel 6, jedoch wurde dem eingesetzten $C_4$-Gemisch Diisobuten im Gewichtsverhältnis 0,7:1 (Beispiel 8) und 1:1 (Beispiel 9) zugemischt. Die Zugabe von Methanol, bezogen auf eingesetztes $C_4$-Gemisch, betrug $2 \times 10^3$ ppm. Beispiel 7 wurde in entsprechender Weise durchgeführt, jedoch ohne Zugabe von Diisobuten. Die Einsatz- und Endprodukte und sonstige Kenndaten der Reaktionen sind aus Tabelle 2 ersichtlich.

<u>EC 118</u>

0048893

Tabelle 2

| Beispiel | [Nr] | 7 | 8 | 9 |
|---|---|---|---|---|
| **Einsatzgemisch:** | | | | |
| $C_4$ | [g] | 60,0 | 60,0 | 60,0 |
| Diisobuten | [g] | <0,1 | 43,0 | 60,0 |
| **Reaktionsprodukt:** | | | | |
| $C_4$ | [g] | 25,4 | 25,3 | 24,8 |
| $C_8$ | [g] | 19,9 | 55,9 | 66,6 |
| $C_{12}$ | [g] | 10,9 | 18,5 | 25,3 |
| $C_{16}$ | [g] | 3,3 | 3,2 | 3,2 |
| $C_{20}$ | [g] | 0,2 | 0,1 | 0,1 |
| **Isomerisierungsgrad** Buten-(1) zu Buten-(2) | [%] | 35,2 | 33,6 | 34,2 |
| Codi II in $C_8$ | [%] | 18,4 | 17,2 | 15,8 |

Codi II hat die bei Tabelle 1 angegebene Bedeutung.

EC 118

Wie aus der Tabelle 2 ersichtlich ist, nimmt der $C_{12}$-Anteil durch Zugabe von Diisobuten selektiv zu. Die Aufschlüsselung des $C_4$- und $C_8$-Bereiches bestätigte die in der Tabelle 1 aufgeführten Werte hinsichtlich der Unterdrückung der Doppelbindung-Isomerisierung von Buten-(1) und die Verminderung der Codimeren II im $C_8$-Bereich.

EC 118

Patentansprüche

1. Verfahren zur Herstellung von $C_4$-Oligomeren und Alkyl-tert.-butylethern durch Umsetzung von Isobuten und Alkanolen in Gegenwart von Kationenaustauschern, dadurch gekennzeichnet, daß man die Umsetzung von Isobuten zu $C_4$-Oligomeren und die Umsetzung von Isobuten mit Alkanolen zu Alkyl-tert.-butylethern gleichzeitig in einem Reaktor in flüssiger Phase bei 20 bis 140°C und in Gegenwart eines makroporösen oder gelförmigen, sauren Kationenaustauschers in der $H^+$-Form durchführt, der 0,05 bis 5 g eines oder mehrerer Metalle der 7. und/oder 8. Nebengruppe des Periodensystems der Elemente in elementarer Form pro Liter trockenen Kationenaustauscher, enthält, wobei der Kationenaustauscher einen Vernetzungsgrad im Bereich 2 bis 65 % und eine spezifische Oberfläche im Bereich von 5 bis 750 m²/g trockenes Austauscherharz aufweist.

2. Verfahren nach Anspruch 1, dadurch gekennzeichnet, daß der Kationenaustauscher als Metall der 7. und/oder 8. Nebengruppe Palladium, Platin, Rhenium oder Nickel jeweils für sich alleine enthält.

3. Verfahren nach Ansprüchen 1 und 2, dadurch gekennzeichnet, daß das Metall in ionischer Form auf dem Kationenaustauscher abgeschieden und mit Wasserstoff in die elementare Form überführt wird.

EC 118

4. Verfahren nach Anspruch 3, dadurch gekennzeichnet, daß die Überführung des Metalls in die elementare Form bei 50 bis 150°C und einem Wasserstoffdruck von 2 bis 50 bar durchgeführt wird.

5. Verfahren nach Ansprüchen 1 bis 4, dadurch gekennzeichnet, daß das Molverhältnis von Isobuten zu Alkanol im Bereich von 0,5 bis 5 x $10^5$ zu 1 liegt.

6. Verfahren nach Ansprüchen 1 bis 5, dadurch gekennzeichnet, daß man die Oligomerisierung und Veretherung bei einer Belastung des Kationenaustauschers von a (wie in der Beschreibung definiert) von 0,1 bis 10 durchführt.

7. Verfahren nach Ansprüchen 1 bis 6, dadurch gekennzeichnet, daß man die Oligomerisierung und Veretherung bei einer Temperatur im Bereich 20 bis 120°C durchführt.

8. Verfahren nach Ansprüchen 1 bis 7, dadurch gekennzeichnet, daß man Isobuten in Form von Gemischen einsetzt, die beim thermischen oder katalytischen Cracken von $C_4$-Schnitten erhalten werden.

9. Verfahren nach Ansprüchen 1 bis 8, dadurch gekennzeichnet, daß man als Alkanol Methanol einsetzt.

EC 118

10. Verfahren nach Ansprüchen 1 bis 9, dadurch gekennzeichnet, daß man das Reaktionsprodukt im Gewichtsverhältnis von Rückführung zu Entnahme von 0,1 bis 10:1 in die Reaktion zurückführt.

EC 118

**Europäisches Patentamt**

**EUROPÄISCHER RECHERCHENBERICHT**

0048893

Nummer der Anmeldung

EP 81 10 7314.7

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A,P | EP - A1 - 0 022 509 (BASF) <br> * Ansprüche 1, 2, 5 * <br> -- | 1,9 |
| A | FR - A - 1 583 594 (MOBIL OIL) <br> * Ansprüche 1, 4, 11 * <br> -- | 1,2 |
| A | FR - A1 - 2 312 483 (ENTREPRISE DE RECHERCHES ET D'ACTIVITES PETROLIERES) <br> * Anspruch 1 * <br> -- | 1 |
| A | FR - A1 - 2 444 021 (CHEMISCHE WERKE HULS) <br> * Anspruch 1 * <br> -- | 1 |
| A | US - A - 3 954 883 (W.O. HAAG et al.) <br> * Ansprüche 1, 2 * <br> ---- | 1,2 |

**KLASSIFIKATION DER ANMELDUNG (Int. Cl.³)**

C 07 C   2/24
C 07 C 41/06
C 07 C 43/04
B 01 J 31/28
B 01 J 31/32

**RECHERCHIERTE SACHGEBIETE (Int. Cl.³)**

B 01 J 31/08
B 01 J 31/10
B 01 J 31/28
B 01 J 31/32
C 07 C   2/08
C 07 C   2/24
C 07 C 41/01
C 07 C 41/06
C 07 C 43/04

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X | Der vorliegende Recherchenbericht wurde für alle Patentansprüche erstellt.

| Recherchenort | Abschlußdatum der Recherche | Prüfer |
|---|---|---|
| Berlin | 21-12-1981 | KNAACK |

EPA form 1503.1   06.78